# EUROPEAN PATENT APPLICATION

(11) **EP 4 349 195 A1**
(43) Date of publication of application: **10.04.2024**
(21) Application number: 23798868.8
(22) Date of filing: 02.06.2023
(51) Int. Cl.: A24F 40/50, G06Q 50/10, G01R 27/26, G01R 19/165, G01R 19/25, A24F 40/40, A24F 40/51, A24F 40/65, A24F 40/46

(54) **APPARATUS AND METHOD FOR IMPLEMENTING DIGITAL TASTE AND DIGITAL SCENT**

(30) Priority: 10.08.2022 KR 20220100075
(71) Applicant: KT & G Corporation, Daejeon 34337 (KR)
(72) Inventor: JEONG, Minseok, Daejeon 34128 (KR); KIM, Jae Hyun, Daejeon 34128 (KR); CHUNG, Tae Young, Daejeon 34128 (KR)
(74) Representative: Ter Meer Steinmeister & Partner
(86) International application number: PCT/KR2023/007596
(87) International publication number: WO 2024/034804

(57) **Abstract**

A method of implementing digital cigarette flavor and digital cigarette flavor is provided, and more particularly, a method of providing a user who uses a digital cigarette to smoke a digital cigarette using a digital recipe. A method of controlling an electronic device provides receiving a digital recipe, determining whether a first area of a mouthpiece unit is in contact with a first portion of an object, determining whether a second area of the mouthpiece unit is in contact with a second portion of the object, determining whether a first terminal and a second terminal of the mouthpiece unit are in contact with a third portion of the object, and based on the contact of the first terminal and the second terminal of the mouthpiece unit with the third portion of the object, applying a current corresponding to the digital recipe to the third portion of the object.

## Description

This application claims the benefit of Korean Patent Application No. 10-2022-0100075 filed on August 10, 2022, in the Korean Intellectual Property Office, the entire disclosure of which is incorporated herein by reference for all purposes.

### BACKGROUND

### 1. Field of the Invention

The following embodiments relate to a method of implementing digital taste and digital flavor and, more particularly, a method of providing various digital recipes to a user of a digital cigarette and providing taste and flavor corresponding to a recipe selected by the user.

### 2. Description of the Related Art

The demand for electronic cigarettes, or e-cigarettes, has recently been on the rise. The rising demand for e-cigarettes has accelerated the continued development of e-cigarette-related functions. The e-cigarette-related functions may include, in particular, functions according to the types and characteristics of e-cigarettes.

### SUMMARY

Embodiments provide various digital recipes to a user.

Embodiments provide taste and flavor only with an electronic device without a stick or a cigarette by digitally implementing the taste and flavor of a cigarette.

According to one embodiment, a method of controlling an electronic device, the method includes receiving a digital recipe, determining whether a first area of a mouthpiece unit is in contact with a first portion of an object, determining whether a second area of the mouthpiece unit is in contact with a second portion of the object, determining whether a first terminal and a second terminal of the mouthpiece unit are in contact with a third portion of the object, and based on the contact of the first terminal and the second terminal of the mouthpiece unit with the third portion of the object, applying a current corresponding to the digital recipe to the third portion of the object.

The method further includes heating a nicotine medium and a heating wire of the mouthpiece unit based on the received digital recipe.

The determining of the contact of the first portion of the object includes determining whether a capacitance value of the first area of the mouthpiece unit exceeds a first capacitance threshold value, and the determining of the contact of the second portion of the object includes determining whether a capacitance value of the second area of the mouthpiece unit exceeds a second capacitance threshold value.

The determining of the contact of the third portion of the object includes measuring a voltage of the second terminal of the mouthpiece unit, and when the measured voltage exceeds a third voltage threshold value, determining that the mouthpiece unit is in contact with the third portion of the object.

The applying of the current to the third portion of the object includes applying the current to the third portion of the object only when the first terminal and the second terminal of the mouthpiece unit are in contact with the third portion of the object.

According to one embodiment, an electronic device includes a main body, a mouthpiece unit detachable from the main body, wherein the main body includes a communication unit configured to receive a digital recipe, and a processor configured to determine whether a first area of the mouthpiece unit is in contact with a first portion of an object, determine whether a second area of the mouthpiece unit is in contact with a second portion of the object, determine whether a first terminal and a second terminal of the mouthpiece unit are in contact with a third portion of the object, and based on the contact of the first terminal and the second terminal of the mouthpiece unit with the third portion of the object, apply a current corresponding to the digital recipe to the third portion of the object.

The mouthpiece unit includes an aerosol intake through which aerosol inhaled by the object passes.

The electronic device further includes a first terminal and a second terminal in a third area of the electronic device, and a heating wire unit including a heating wire disposed in a third area of the mouthpiece unit and heating the mouthpiece unit, based on the digital recipe.

Embodiments may provide various digital recipes to a user.

Embodiments may provide taste and flavor only with an electronic device without a stick or a cigarette by digitally implementing the taste and flavor of a cigarette.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1A is a block diagram illustrating an electronic device according to one embodiment;
FIG. 1B schematically illustrates an example of a part in which a tongue of a user contacts a mouthpiece unit according to one embodiment;
FIG. 2 illustrates a flowchart of a control method of an electronic device according to one embodiment;
FIG. 3A illustrates a flowchart of a method of an electronic device to determine whether a mouthpiece unit is in contact with an object according to one embodiment;
FIG. 3B illustrates a flowchart of an operating method of a processor to update a resistance value of an object;
FIG. 4A schematically illustrates a configuration of a mouthpiece according to one embodiment;
FIG. 4B schematically illustrates a cross-section of a mouthpiece according to one embodiment;
FIG. 4C schematically illustrates a cross-section of a mouthpiece configured in a multi-layer flexible printed circuit board (FPCB) according to one embodiment;
FIG. 5 illustrates a flowchart of a control method of an electronic device according to one embodiment;
FIG. 6A schematically illustrates an aerosol generating device and a flavor diffusing cartridge of an electronic cigarette device;
FIG. 6B illustrates a flowchart of a flavoring method of an electronic cigarette device according to one embodiment;
FIG. 6C illustrates a flowchart of a method of an electronic cigarette device to determine whether to spray aerosolized flavor;
FIG. 7A schematically illustrates an operation of a flavor sensor of an electronic device according to one embodiment;
FIG. 7B illustrates a data distribution diagram based on a flavor analyzed by an electronic device according to one embodiment;
FIG. 7C illustrates an example of showing data of a sensing result of a flavor material by an electronic device according to one embodiment;
FIG. 7D illustrates a flowchart of an electronic device to generate a flavor recipe according to one embodiment;
FIG. 8 illustrates a flowchart of an operating method of a terminal according to one embodiment;
FIG. 9A schematically illustrates a configuration of an electronic device according to one embodiment;
FIG. 9B illustrates a flowchart of an operating method of an electronic device as a digital cigarette according to one embodiment; and
FIG. 9C illustrates a flowchart of an operating method of a terminal for a digital cigarette according to one embodiment.

### DETAILED DESCRIPTION

The following detailed structural or functional description is provided as an example only and various alterations and modifications may be made to the examples. Here, the examples are not construed as limited to the disclosure and should be understood to include all changes, equivalents, and replacements within the idea and the technical scope of the disclosure.

Terms, such as first, second, and the like, may be used herein to describe components. Each of these terminologies is not used to define an essence, order or sequence of a corresponding component but used merely to distinguish the corresponding component from other component(s). For example, a first component may be referred to as a second component, and similarly the second component may also be referred to as the first component.

It should be noted that if one component is described as being "connected", "coupled", or "joined" to another component, a third component may be "connected", "coupled", and "joined" between the first and second components, although the first component may be directly connected, coupled, or joined to the second component.

The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

Hereinafter, examples will be described in detail with reference to the accompanying drawings. When describing the embodiments with reference to the accompanying drawings, like reference numerals refer to like elements and a repeated description related thereto will be omitted.

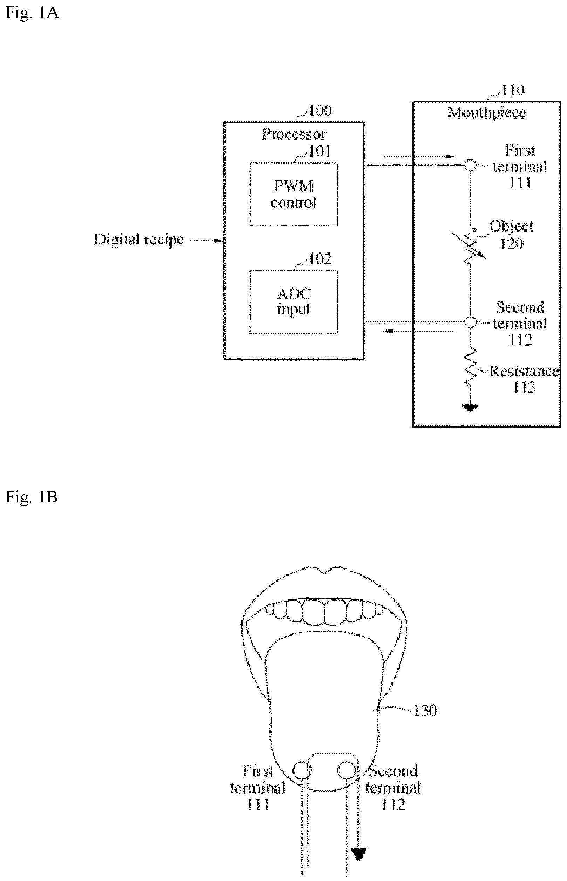

FIG. 1A is a block diagram illustrating an electronic device according to one embodiment.

Referring to FIG. 1A, an electronic device in one embodiment may be referred to as an aerosol generating device, an electronic cigarette device, or a smoking stick. "Smoking" may indicate heating, burning, and other actions intended to cause the release of smoke or aerosol from a substance that can be smoked.

The electronic device in one embodiment may include a processor 100 and a mouthpiece unit 110. The processor 100 may include a PWM (pulse width modulation) control unit 101 and an analog-to-digital converter (ADC) input terminal 102. The mouthpiece unit 110 may include a second terminal 112, a resistor 113, and a first terminal 111 contacting an object 120 shown as variable resistance.

A conventional smoking product provides a smoking action by lighting and burning a cigarette and smoking the cigarette or heating a stick or liquid using a device. However, a cigarette may require a separate combustion tool (e.g., a lighter), inconvenience may be caused by ash generated during smoking, and an issue related to harmfulness may occur. A cigarette-type electronic cigarette may require inserting a stick before smoking and a byproduct, which is a cigarette end, may be generated after finishing smoking. A liquid-type electronic cigarette may provide a uniform amount of atomization and smoking taste but may require replacing a corresponding cartridge every time to feel various tastes and flavors of liquid cigarettes.

The electronic device in one embodiment may digitally implement the taste and/or flavor of a cigarette and may provide the taste and flavor to a user only with the electronic device without a stick or a cigarette. More particularly, the electronic device may receive a digital recipe from a terminal (e.g., a smartphone) of a user and may provide a digital taste and digital flavor corresponding to the digital recipe to the user.

The digital taste in one embodiment may be implemented by applying a microcurrent to the taste cells of a user and the digital flavor may be implemented by combining flavor materials using a plurality of liquid cartridges included in the electronic device. The digital recipe in one embodiment may include a digital taste recipe and a digital flavor recipe, the digital taste recipe may be a method of digitally implementing a predetermined taste, and the digital flavor recipe may be a method of digitally implementing a predetermined flavor.

The processor 100 in one embodiment may apply a microcurrent that implements a digital taste in the electronic device based on the received digital recipe. A detailed description of a method of implementing digital taste and flavor is provided below with reference to FIGS. 1B to 9B.

The PWM controller 101 of the processor 100 in one embodiment may apply a microcurrent to the mouthpiece unit 110 in a PWM method. PWM control may be a voltage control method in which as much power as required by an output is supplied from an input by turning on a switch at a predetermined cycle. Accordingly, depending on the required output power, an on-and-off ratio and a duty cycle may vary. As a PWM control method, a method of applying a microcurrent to the mouthpiece unit 110 is described below.

The ADC input terminal 102 of the processor 100 in one embodiment may convert a voltage value measured by the second terminal 112 into a digital signal. The processor 100 may determine a resistance value of the object 120 based on the voltage value converted into the digital signal.

The object 120, displayed as variable resistance in one embodiment, may include the lips and tongue of a user, but the object 120 is not limited thereto. Hereinafter, for ease of description, a first portion of the object 120 may be assumed to be an upper lip of a user and a second portion of the object 120 may be assumed to be a lower lip of the user, and a third portion of the object 120 may be referred to as a tongue of the user.

FIG. 1B schematically illustrates an example of a part in which a tongue of a user contacts a mouthpiece unit according to one embodiment.

The description provided with reference to FIG. 1A may apply to the description provided with reference to FIG. 1B, and any repeated description related thereto is omitted.

A tongue 130 of a user in one embodiment may include a taste receptor (e.g., a neuron). The taste receptor may generate a taste substance for feeling the taste by causing chemical reaction with a substance touching the tongue 130 of the user and may transmit, to the brain, an electrical signal that enables to distinguish the taste of sweet, salty, and bitter depending on the concentration of the generated taste substance, and thereby, the user perceives the taste.

The tongue 130 of the user in one embodiment may contact the first terminal 111 and the second terminal 112 of the mouthpiece unit 110. Because an insulator is formed between the first terminal 111 and the second terminal 112, a circuit may not be connected in a normal state. When the tongue 130 of the user contacts the mouthpiece, the tongue 130 of the user may be modeled as resistance between the first terminal 111 and the second terminal 112.

The mouthpiece unit 110 may sense contact with the tongue 130 of the user. When the first terminal 111 and the second terminal 112 of the mouthpiece unit 110 are in contact with the tongue 130 of the user, an insulated gap between the first terminal 111 and the second terminal 112 may be connected by the tongue 130 of the user. When the circuit is connected, the tongue 130 of the user may be regarded as variable resistance and a current may flow between the first terminal 111 and the second terminal 112. The mouthpiece unit 110 may measure a voltage of the second terminal 112 when the mouthpiece unit 110 contacts the tongue 130 of the user. When the voltage exceeds a predetermined value (hereinafter, referred to as a first threshold value), the processor 100 may determine that the tongue 130 of the user is in contact with the mouthpiece unit 110.

The voltage measured at the second terminal 112 in one embodiment may vary depending on a state of the tongue 130 of the user. When a resistance value of the tongue 130 of the user changes, the voltage value measured at the second terminal 112 may change. For example, because each user's tongue may have a different thickness, a resistance value of the tongue may be different for each user. Because a contact position in which a tongue of a user is in contact with the mouthpiece may vary depending on a user's habit, a resistance value of the tongue may vary for each user. Because saliva concentration and pH may vary, a resistance value of the tongue may vary for each user.

Even in the case of the same user's tongue, a resistance value may vary depending on the state of the user. For example, when comparing cases before and after a user drinks a beverage, a current flow through the tongue of the user may vary, and thus, a resistance value may change even in the case of the same user's tongue. Furthermore, even in the case of the same user's tongue, a resistance value may vary depending on a contact position with the mouthpiece unit 110 or a health condition.

The processor 100 in one embodiment may determine a resistance value of the tongue 130 of the user based on the voltage value measured at the second terminal 112. When the resistance value of the tongue 130 of the user is determined, the processor 100 may apply a microcurrent to the mouthpiece unit 110 based on the received digital recipe. The PWM controller 101 may determine a microcurrent value based on a resistance value of an object in the digital recipe based on the resistance value of the object. The determined microcurrent value may be applied to the tongue 130 of the user through the first terminal 111 and the second terminal 112.

The resistor 113 in one embodiment may be connected and grounded to the second terminal 112. Depending on the size of the resistor 113, an intensity of a current applied to the tongue 130 of the user may be determined. Because the human tongue has a relatively small resistance value, the resistor 113 may be designed to have a large value to generate a microcurrent (e.g., tens of uA to hundreds of uA).

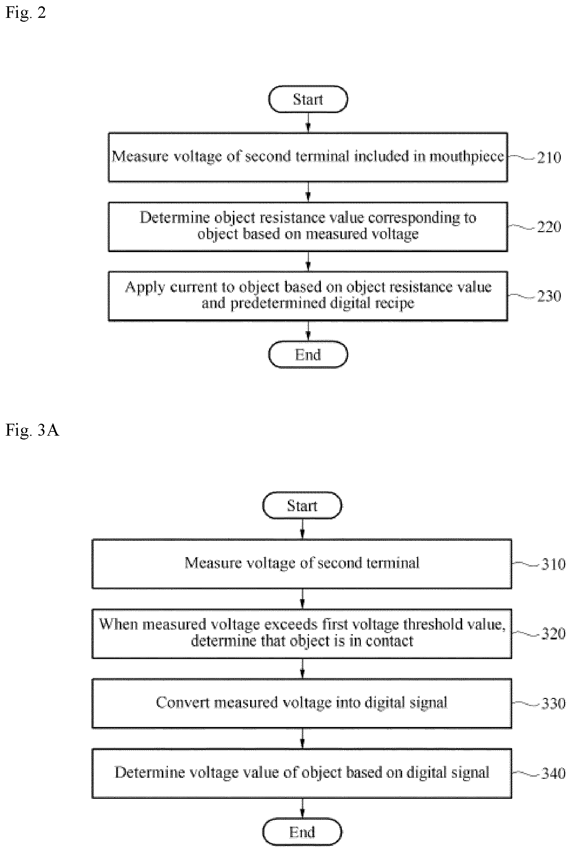

FIG. 2 illustrates a flowchart of a control method of an electronic device according to one embodiment.

Operations 210 to 230 may be performed by a processor described with reference to FIGS. 1A and 1B, the description of FIGS. 1A and 1B may be applicable to the description of FIG. 2, and a repeated description thereof may be omitted.

In operation 210, an electronic device in one embodiment may measure a voltage of a second terminal included in a mouthpiece.

In one embodiment, when the object 120 contacts the first terminal 111 and the second terminal 112, a circuit including the first terminal and the second terminal may be switched from an open circuit to a connected circuit through which a current may flow through the object. Accordingly, a voltage of the second terminal may be measured as voltage distribution occurs based on the resistance of the modeled object 120 and the resistor 113. The mouthpiece unit 110 may measure and transmit the voltage of the second terminal to the processor 100.

In operation 220, the electronic device in one embodiment may determine an object resistance value corresponding to the object based on the measured voltage.

The processor 100 in one embodiment may convert the measured voltage value into a digital signal through the ADC input terminal 102. The processor 100 may determine a resistance value of the object based on the digital signal. For example, because a power value of a battery and the resistor 113 are predetermined in the electronic device, when the tongue 130 of the user contacts the mouthpiece unit 110, the processor 100 may calculate the resistance value of the tongue 130 of the user by voltage distribution between the resistance of the tongue 130 of the user and the resistor 113 of the circuit between the first terminal 111 and the second terminal 112.

In operation 230, the electronic device in one embodiment may apply a current to the object based on the object resistance value and the predetermined digital recipe.

The processor 100 in one embodiment may determine power capable of applying a current corresponding to the digital recipe based on the determined resistance value. For example, the digital recipe may include data on power to be applied determined based on the resistance value of the tongue 130 of the user by digitizing predetermined taste and flavor. Accordingly, when the resistance value of the tongue 130 of the user is determined, the processor 100 may apply a power value corresponding to the resistance value of the tongue 130 of the user in the digital recipe through the PWM controller 101.

FIG. 3A illustrates a flowchart of a method of an electronic device to determine whether a mouthpiece unit is in contact with an object according to one embodiment.

Operations 310 to 340 may be performed by a processor described with reference to FIGS. 1A and 1B, the description of FIGS. 1A to 2 may be applicable to the description of FIG. 3A, and a repeated description thereof may be omitted.

In operation 310, an electronic device in one embodiment may measure the voltage of a second terminal.

In operation 320, the electronic device in one embodiment may determine that an object is in contact when the measured voltage exceeds a first voltage threshold value.

In operation 330, the electronic device in one embodiment may convert the measured voltage into a digital signal.

In operation 340, the electronic device in one embodiment may determine a resistance value of the object based on the digital signal.

According to one embodiment, the electronic device may determine whether the electronic device is in contact with the object 120 based on the measured voltage.

For example, when an open circuit is connected as the tongue 130 of the user contacts the first terminal 111 and the second terminal 112, the voltage of the second terminal 112 may be measured. The processor 100 may determine that the tongue 130 of the user is in contact with the mouthpiece unit 110 when the voltage value measured at the second terminal 112 exceeds the first voltage threshold value preset to the processor 100.

The processor 100 may convert the measured voltage value into a digital signal through the ADC input terminal 102.

The processor 100 may determine the resistance value of the object corresponding to the digital signal converted through the ADC input terminal 102.

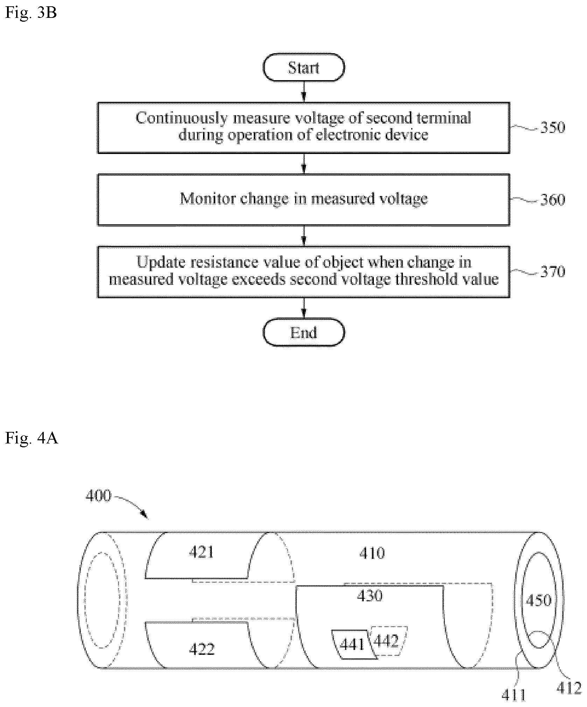

FIG. 3B illustrates a flowchart of an operating method of a processor to update a resistance value of an object.

Operations 350 to 370 may be performed by a processor described with reference to FIGS. 1A and 1B, the description of FIGS. 1A to 3A may be applicable to the description of FIG. 3B, and a repeated description thereof may be omitted.

In operation 350, the processor in one embodiment may continuously measure the voltage of the second terminal during the operation of the electronic device.

In operation 360, the processor in one embodiment may monitor a change in the measured voltage.

In operation 370, the processor in one embodiment may update the resistance value of the object when the change in the measured voltage exceeds a second voltage threshold value.

According to one embodiment, in the electronic device, the received digital recipe may include data for applying an appropriate microcurrent based on the resistance value of the object 120. Since the resistance value of the object 120 may vary in real time, the electronic device may need to continuously monitor the resistance value of the object 120. For the electronic device to accurately implement a digital taste required by the digital recipe in the object 120, precise control of a microcurrent based on the resistance value of the object 120 may be required, and when the resistance value of the object changes more than a predetermined level, the processor 100 may again determine the power of the PWM controller 101 to adjust an intensity of the microcurrent.

The processor 100 in one embodiment may update the resistance value of the object when the change in the measured voltage exceeds a second voltage threshold value. For example, as the state of the tongue 130 of the user changes while the user smokes, the voltage value of the second terminal 112 may change. By comparing the changed voltage value of the second terminal 112 with the first voltage threshold value, when an absolute value of the changed voltage value changes by more than the second threshold value, the processor 100 may perform an operation to update the resistance value of the object. The change in the voltage value measured at the second terminal 112 may indicate a change in the resistance value of the tongue 130 of the user and the processor 100 may update to a new resistance value of the object corresponding to the digital recipe. The processor 100 may perform an operation to apply a microcurrent to the tongue 130 of the user through the PWM controller 101 based on the new resistance value of the object.

FIG. 4A schematically illustrates a configuration of a mouthpiece according to one embodiment.

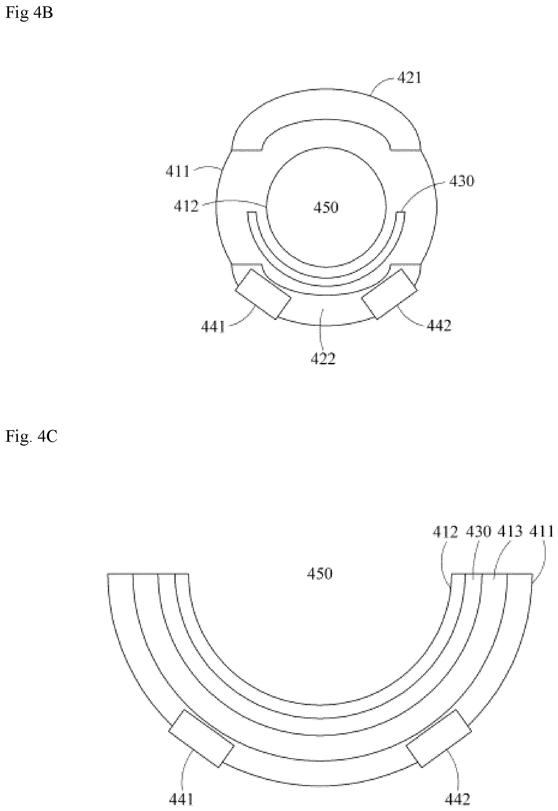

FIG. 4B schematically illustrates a cross-section of a mouthpiece according to one embodiment.

FIG. 4C schematically illustrates a cross-section of a mouthpiece configured in a multi-layer flexible printed circuit board (FPCB) according to one embodiment.

The description provided with reference to FIGS. 1A to 3B may also apply to the descriptions provided with reference to FIGS. 4A to 4C, and any repeated description related thereto will be omitted. In addition, all operations of a mouthpiece may be controlled by the processor 100 described with reference to FIGS. 1A and 3B.

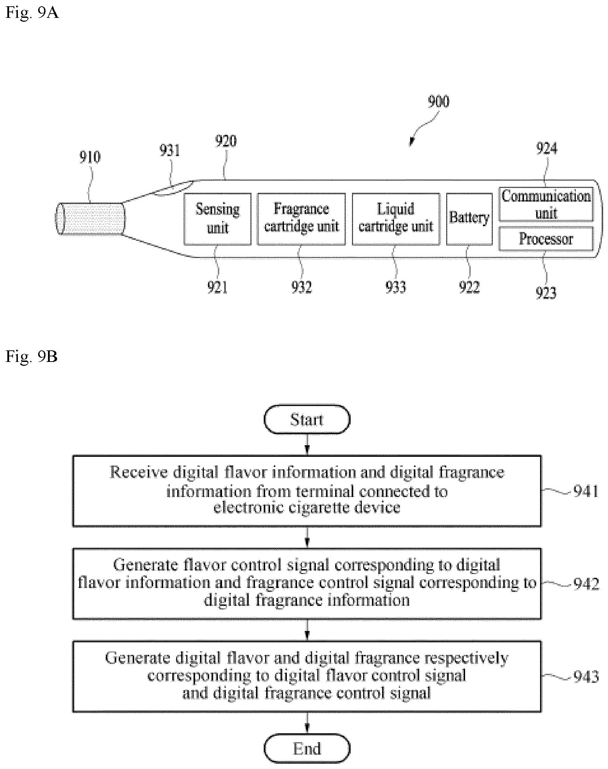

An electronic device in one embodiment may include a main body and a mouthpiece unit 400. The mouthpiece unit 400 may be detachable from the main body. Although not shown in FIG. 4, the main body may be a portion except for the mouthpiece unit 400 in an electronic cigarette device. Hereinafter, a detailed description of the main body is provided with reference to FIG. 9A.

The mouthpiece unit 400 in one embodiment may include a body 410 having a cylindrical shape and similar size and intensity to a typical cigarette, an outer circumferential surface 411, an inner circumferential surface 412, a first capacitor sensor 421, and a second capacitor sensor 422 for sensing contact with lips, and a first terminal 441 and a second terminal 442 applying a microcurrent to implement a digital taste.

The mouthpiece unit 400 in one embodiment may include a heating wire unit 430 designed in a thermal resistance pattern and an aerosol intake 450 allowing a user to inhale aerosol.

The body 410 of the mouthpiece unit in one embodiment may have a cylindrical shape similar to a typical cigarette. The body 410 may need to be longer than a filter (approximately 30 mm) of a stick or a cigarette. The filter of a stick or cigarette may be a filter of an electronic cigarette in which a typical cigarette or a stick is put and used. When a user bites the mouthpiece unit 400 to inhale smoke, only the lips of the user may be in contact with the mouthpiece 400 if the filter has a length that is the same as the length of the typical cigarette or stick. Accordingly, there is a need to provide a longer structure of the body 410 of the mouthpiece unit than a typical cigarette or a stick such that the tongue of the user may naturally contact. However, the shape of the body 410 in the present disclosure is not limited to the shape, thickness, or length of the typical cigarette described above.

In one embodiment, a proximal end may be a direction close to a side in contact with the tongue of the user in the body 410 of the mouthpiece unit. On the other hand, a distal end may be a direction in which the body 410 of the mouthpiece is inserted into the main body.

The first capacitor sensor 421 in one embodiment may be disposed in a first area of the outer circumferential surface of the mouthpiece unit 400.

The second capacitor sensor 422 in one embodiment may be disposed in a second area of the outer circumferential surface spaced apart in a circumferential direction of the first area.

The first microcurrent applying terminal 441 in one embodiment may be disposed in a third area of the outer circumferential surface spaced apart in a longitudinal direction of the second area. The first microcurrent applying terminal 441 may be disposed in the third area in a proximal end direction based on the second area. In addition, the third area may be spaced apart from the second area by a predetermined distance (e.g., more than 10 mm) in the longitudinal direction. The first microcurrent applying terminal 441 may include the first terminal 111 described above.

The second microcurrent applying terminal 442 in one embodiment may be disposed in a fourth area of the outer circumferential surface spaced apart in a circumferential direction of the third area. The second microcurrent applying terminal 442 may include the second terminal 112 described above.

The heating wire unit 430 in one embodiment may be disposed on a fifth area in a space between the outer circumferential surface 411 and the inner circumferential surface 412. The heating wire unit 430 may heat the mouthpiece unit 400 or an aerosolized medium similar to a temperature of mainstream smoke (smoke directly sucked into the mouth of a smoker when smoking and sidestream smoke refers to smoke that is not) of a typical cigarette or stick. For example, because the temperature of mainstream smoke of a typical cigarette or stick is approximately 25 °C to approximately 55 °C, the heating wire unit 430 may be designed a thermal resistance value and pattern to be similar to the temperature of the typical cigarette or stick. However, the temperature heated by the heating wire unit 430 in the present disclosure is not limited to the temperature between 25 °C and 55 °C.

The insulating portion 413 may be disposed between the outer circumferential surface 411 and the heating wire unit 430. The heating wire unit 430 may be designed in a thermal resistance pattern that is able to apply thermal stimulation. When the heating wire unit 430, the first microcurrent applying terminal 441, and the second microcurrent applying terminal 442 are not insulated, the heating wire unit 430 may be connected to the microcurrent applying terminals through the tongue and thereby a resistance value may change and the heating temperature of thermal resistance or the intensity of microcurrent may change. That is, when resistance and thermal resistance of a microcurrent circuit are shorted through the tongue, normal operation of the circuit may be impaired. Accordingly, the heating wire unit 430, the first microcurrent terminal 441, and the second microcurrent terminal 442 may be open through the insulating portion 413.

The aerosol intake 450 in one embodiment may be formed inside the mouthpiece unit 400 and may allow the discharge of aerosol. Depending on the selection of a user, the main body may aerosolize and provide nicotine or non-nicotine medium to the user through the mouthpiece unit 400.

The mouthpiece unit 400 in one embodiment is inserted into the main body and operated. Although not shown in FIGS. 4A to 4C, in a direction of the distal end of the mouthpiece unit 400, an insertion detection sensor may be disposed, or in a direction of an insertion portion of the main body, the insertion detection sensor of the mouthpiece unit 400 may be disposed.

In one embodiment, the first capacitor sensor 421 may sense whether an upper lip of a user is in contact with the mouthpiece unit 400 and the second capacitor sensor 422 may sense whether a lower lip of a user is in contact with the mouthpiece unit 400. The first capacitor sensor and the second capacitor sensor may transmit sensed information to the processor 100. The processor 100 may control operations of an electronic device, may control the application of microcurrent, and may analyze a smoking pattern of a user, based on the sensed information.

The mouthpiece unit 400 in one embodiment may be configured in a circuit including the heating wire unit 430 and a circuit including microcurrent applying terminals (e.g., the first microcurrent applying terminal and the second microcurrent applying terminal). The circuits may be configured in a multi-layer FPCB structure in which pattern designs of the circuits are divided into a pattern design of the circuit including the heating wire unit 430 and a pattern design of the circuit including the microcurrent applying terminals and a pattern of each layer is divided with an insulating layer. Unlike a typical PCB structure, the FPCB structure may be a FPCB to be applied to surround the mouthpiece unit 400 having a shape of a cylindrical cigarette or a stick. Through the mouthpiece unit 400 to which the multi-layer FPCB structure is applied, a user may feel less of difference compared to smoking through a typical cigarette or a stick.

In one embodiment, the first microcurrent applying terminal 441 and the second microcurrent applying terminal 442 may allow the tongue 130 of the user to contact the terminals as shown in FIG. 1B and FIGS. 4A to 4C. FIG. 4C illustrates a portion including the heating wire unit 430 and the microcurrent applying terminals of upper and lower portions of a cross-section of the mouthpiece. Because generally, the tongue 130 of the user is able to contact only one of upper and lower portions of an object having a cylindrical shape, microcurrent applying terminals may need to be disposed both the upper and lower portions such that all of the microcurrent applying terminals are in contact with the tongue 130 of the user.

The main body in one embodiment may include a processor and a communication unit receiving a digital recipe. The processor 100 in one embodiment may determine whether a first area of the mouthpiece unit is in contact with a first portion of an object. The processor 100 may determine whether a second area of the mouthpiece unit is in contact with a second portion of the object. The processor 100 may determine whether the first terminal and the second terminal are in contact with a third portion of the object. Based on the contact of the first terminal and the second terminal of the mouthpiece unit with the third portion of the object, the processor 100 may apply a current corresponding to a digital recipe to the third portion of the object. The communication unit in one embodiment is described with reference to FIG. 9A.

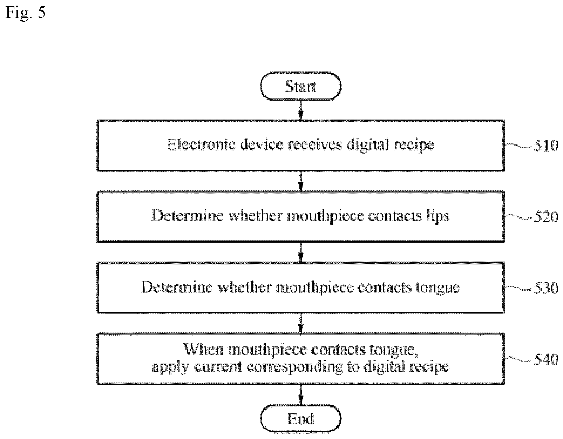

FIG. 5 illustrates a flowchart of a control method of an electronic device according to one embodiment.

Referring to FIG. 5, operations 510 to 540 may be performed by a processor described with reference to FIGS. 1A to 4C, the description of FIGS. 1A to 3C may be applicable to the description of FIG. 5, and a repeated description thereof may be omitted.

In operation 510, an electronic device in one embodiment may receive a digital recipe. The electronic device may receive a digital taste recipe and/or a digital flavor recipe included in the digital recipe from a terminal (e.g., a user terminal connected to the electronic device through a network) of a user.

After operation 510, the heating wire unit 430 of a mouthpiece unit in one embodiment may preheat the electronic device based on the digital recipe. For example, a nicotine medium or a non-nicotine medium embedded in the electronic device may be preheated based on the digital recipe.

When the heating wire unit 430 of the mouthpiece unit and the nicotine medium or the non-nicotine medium reach a predetermined temperature, the electronic device in one embodiment may notify the user of the completion of preheating through vibration.

In operation 520, the electronic device in one embodiment may determine whether the mouthpiece unit is in contact with the lips.

The electronic device may determine whether a first area of the mouthpiece unit is in contact with a first portion (e.g., an upper lip of a user) of an object. As shown in FIG. 4A, a first capacitor sensor may be disposed in the first area of the mouthpiece unit.

The electronic device may determine whether a second area of the mouthpiece unit is in contact with a second portion (e.g., a lower lip of a user) of the object.

More specifically, the electronic device may determine whether a capacitance value of the first capacitor sensor in the first area of the mouthpiece unit exceeds a first capacitance threshold value. For example, when the user contacts the first area with their upper lip to bite the mouthpiece unit 400, a capacitance value of the first capacitance sensor of the first area may change. When the capacitance value changes and exceeds the first capacitance threshold value, the processor 100 of the electronic device may determine that the upper lip of the user contacts the first area.

The electronic device may determine whether a capacitance value of the second capacitor sensor in the second area of the mouthpiece unit exceeds a second capacitance threshold value. For example, when the user contacts the second area with their lower lip to bite the mouthpiece unit 400, a capacitance value of the second capacitance sensor of the second area may change. When the capacitance value changes and exceeds the second capacitance threshold value, the processor 100 of the electronic device may determine that the lower lip of the user contacts the second area.

In operation 530, the electronic device in one embodiment may determine whether the mouthpiece unit is in contact with a tongue.

The electronic device may determine whether the first terminal 111 and the second terminal 112 of the mouthpiece unit 400 are in contact with a third portion (e.g., the tongue of the user) of the object.

The electronic device may measure a voltage of the second terminal 112 of the mouthpiece unit 400 and when the measured voltage exceeds a third voltage threshold value (e.g., a value the same as the first voltage threshold value of FIG. 1A), the electronic device may determine that the mouthpiece unit contacts the third portion of the object.

In operation 540, the electronic device in one embodiment may apply a current corresponding to the digital recipe to the third portion of the object based on the contact of the first terminal 111 and the second terminal 112 of the mouthpiece unit 400 with the third portion of the object.

The electronic device may apply the current to the third portion of the object only when the first terminal 111 and the second terminal 112 of the mouthpiece unit 400 contact the third portion of the object.

For example, when the user bites and uses the mouthpiece unit 400 of the electronic device with their mouth, the user may hold the mouthpiece unit 400 only with their lips and inhale the aerosol. The tongue of the user may not contact the first terminal 111 and the second terminal 112 of the electronic device and in this case, a microcurrent may not need to be applied. Alternatively, when the user bites and uses the mouthpiece unit 400 of the electronic device with their mouth, the lips of the user may contact the first terminal 111 or the second terminal 112. When the lips of the user contact the first terminal 111 and the second terminal 112, a microcurrent should not be applied. That is, in this case, the electronic device may need to determine whether the tongue 130 of the user is in contact to apply the microcurrent. Typically, because the lips and tongue of a person have different resistance values, when a resistance value determined based on a voltage value measured in the second terminal 112 is a resistance value of the lips, the processor 100 may not apply a microcurrent to the terminals of the mouthpiece unit.

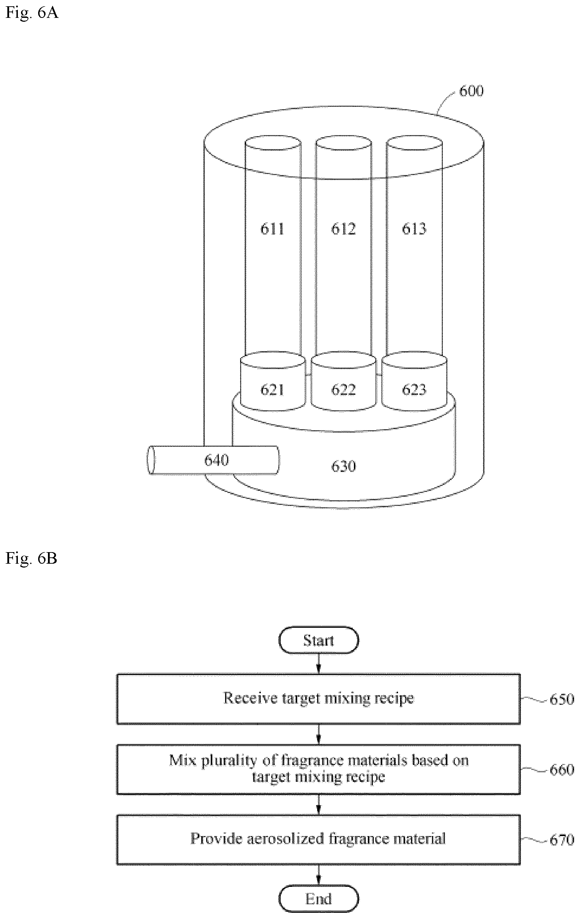

FIG. 6A schematically illustrates an aerosol generating device and a flavor diffusing cartridge of an electronic cigarette device.

The description provided with reference to FIGS. 1A to 5 may also apply to the description provided with reference to FIG. 6A, and any repeated description related thereto will be omitted.

A flavor cartridge unit 600 in one embodiment may include at least one flavor cartridges 611 to 613, flavor material moving valves 621 to 623 respectively attached to the cartridges, a flavor aerosol generating unit 630 for aerosolizing a mixed solution, and a flavor aerosol moving unit 640. The present disclosure describes that the flavor cartridge unit 600 includes three flavor cartridges 611 to 613. However, the number of flavor cartridges is not limited thereto and the flavor cartridge unit 600 may include various numbers of flavor cartridges. Likewise, the number of solution moving valves 621 to 623 in the present disclosure is not limited to three.

The processor 100 in one embodiment may control the flavor cartridge unit based on a target mixing recipe included in the digital recipe. The processor 100 may control operations of the flavor cartridges 611 to 613, the flavor material moving valves 621 to 623, the flavor aerosol generating unit 630, and the flavor aerosol moving unit 640, based on the target mixing recipe. The target mixing recipe may include mixing ratio data of flavor materials included in the flavor cartridges 611 to 613.

The flavor cartridges 611 to 613 in one embodiment may include different flavor materials, respectively. For example, the first flavor cartridge 611 may include a light-based citrus flavor material. The second flavor cartridge 612 may include a green-based herbal-spicy flavor material. The third flavor cartridge 613 may include a heavy-based tobacco flavor material.

Based on the control of the processor 100, the flavor material moving valves 621 to 623 may control a movement amount of flavor materials included in the flavor cartridges 611 to 613 corresponding to the target mixing recipe.

The flavor aerosol generating unit 630 in one embodiment may aerosolize a plurality of moved flavor materials. When a user puffs for smoking, the processor 100 may control the flavor aerosol generating unit 630 to aerosolize and spray a mixed solution included in the flavor aerosol generating unit 630.

The flavor aerosol moving unit 640 in one embodiment may be a moving tube for spraying an aerosolized and mixed flavor material. Because aerosolized flavor needs to be sprayed in response to a puff of the user, the processor 100 may control the flavor aerosol moving unit 640 to spray the aerosolized flavor in response to a puff of the user.

FIG. 6B illustrates a flowchart of a flavoring method of an electronic cigarette device according to one embodiment.

Referring to FIG. 6B, operations 650 to 670 may be performed by the processor 100 described with reference to FIGS. 1A to 3B and a repeated description provided with reference to FIGS. 1A to 6A may be omitted.

In operation 650, an electronic cigarette device in one embodiment may receive a target mixing recipe. A digital recipe in one embodiment may include information related to the target mixing recipe.

In operation 660, the electronic cigarette device in one embodiment may mix a plurality of flavor materials based on the target mixing recipe.

The processor 100 in one embodiment may transmit a control signal including the target mixing recipe to the flavor cartridge unit 600. The flavor cartridges 611 to 613 and the flavor material moving valves 621 to 623 may mix the plurality of flavor materials by moving the flavor materials to the flavor aerosol generating unit 630 based on the control signal. In this case, the processor 100 may determine an emission amount (e.g., an amount of flavor materials moved from the flavor cartridges 611 to 613) of the plurality of flavor materials based on the target mixing recipe.

In operation 670, the electronic cigarette device in one embodiment may provide an aerosolized flavor material.

When the plurality of flavor materials is mixed and aerosolized in the flavor aerosol generating unit 630 in one embodiment, the flavor aerosol may move through the flavor aerosol moving unit 640 and may be sprayed to the outside of the electronic cigarette device. In this case, the processor 100 may control the flavor cartridge unit 600 such that the flavor aerosol is sprayed in response to a puff motion of the user.

In operation 670, the electronic cigarette device in one embodiment may sense a puff of the user.

The electronic cigarette device in one embodiment may include a puff sensor. Although not shown in the drawings, the puff sensor may sense a puff from a user based on various physical changes in an airflow path or airflow channel. For example, the puff sensor 326 may sense the puff from the user based on any one of a temperature change, a flow change, a voltage change, and a pressure change.

In operation 670, the electronic cigarette device in one embodiment may aerosolize the mixed flavor material. For example, the aerosol generating unit 630 may aerosolize a flavor material by synchronizing with a timepoint of sensing a puff of the user.

The aerosol generating unit 630 in one embodiment may heat and aerosolize the mixed flavor material. In this case, the processor 100 may transmit the control signal to the flavor cartridge unit 600 to aerosolize the flavor material in synchronization with a timepoint of sensing a puff of the user.

In operation 670, the electronic cigarette device in one embodiment may spray an aerosolized flavor material.

The flavor cartridge unit 600 in one embodiment may spray the aerosolized flavor material generated by the flavor aerosol generating unit 630 through the flavor aerosol moving unit 640. In this case, when the processor 100 senses a puff of the user by the puff sensor, the processor 100 may spray the flavor material in synchronization with a timepoint of the puff of the user when a part of the user (e.g., lips or nose of the user) is close to the electronic cigarette device. That is, when the lips of the user are in contact with the electronic cigarette or the nose of the user is close to the electronic cigarette and the aerosolized flavor material is sprayed, the flavor may be provided to the user with even a small amount of the flavor material.

In operation 670, the electronic cigarette device in one embodiment may extract a puff interval of the user based on the puff sensing result.

The processor 100 in one embodiment may sense how many seconds the user puffs based on the puff sensing result of the user sensed by the puff sensor. The processor 100 may extract a puff interval of the user based on the sensed result. For example, when a second puff occurs 7 seconds after a first puff, a third puff occurs 8 seconds after the second puff, and a fourth puff occurs 9 seconds after the third puff, the user's puff occurs every 8 seconds, which is an average of 7 seconds, 8 seconds, and 9 seconds. However, a puff interval of the user in the present disclosure is not measured three times but may be measured more than three times.

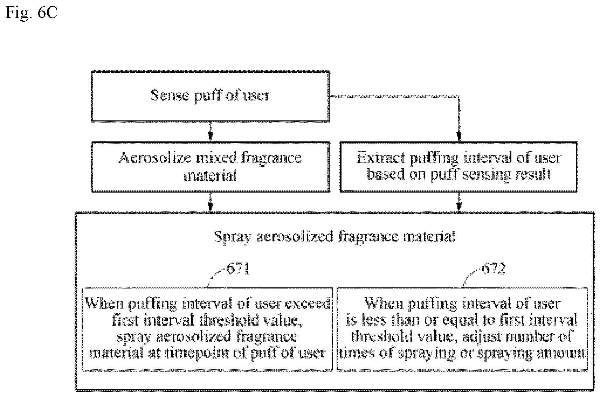

FIG. 6C illustrates a flowchart of a method of an electronic cigarette device to determine whether to spray aerosolized flavor.

Referring to FIG. 6C, operations 671 and 672 may be performed by the processor 100 described with reference to FIGS. 1A to 3.

The description provided with reference to FIG. 6C describes a method in which the processor provides aerosolized flavor in operation 670 of FIG. 6B.

The descriptions provided with reference to FIGS. 1A, 6A, and 6B may be applicable to FIG. 6C and any repeated description related thereto may be omitted.

In operation 671, the electronic cigarette device in one embodiment may spray an aerosolized flavor material at a timepoint of a puff of the user when the puff interval of the user exceeds a first interval threshold.

The processor 100 in one embodiment may determine whether to spray the aerosolized flavor material at a timepoint of a puff of the user by comparing the preset interval threshold with the puff interval of the user. That is, if the user does not feel uncomfortable or the flavor material is not excessively consumed even if the aerosolized flavor material is sprayed at a timepoint of a puff of the user, the processor 100 may instruct the flavor cartridge unit 600 to spray the aerosolized flavor material.

For example, when the preset first interval threshold is 8 seconds and the puff interval of the user (an average value of the first three puff intervals) is determined to be 9 seconds, the processor 100 may instruct the flavor cartridge unit 600 to spray the aerosolized flavor material at every puff of the user.

In operation 672, when the puff interval of the user is less than or equal to the first interval threshold, the electronic cigarette device in one embodiment may adjust the number of sprays or a spray amount.

When the puff interval of the user is less than or equal to the first interval threshold, processor 100 in one embodiment may adjust the number of sprays or a spray amount of the aerosolized flavor material by comparing the preset first interval threshold with the puff interval of the user. That is, when the puff interval of the user is too short, spraying the aerosolized flavor material at every puff of the user may cause discomfort to the user or unnecessary consumption of the flavor material. Accordingly, when the puff interval of the user is less than or equal to the preset first interval threshold, the aerosolized flavor material may be sprayed at some of the user's puffs.

Alternatively, when the puff interval of the user is less than or equal to the preset first interval threshold, the electronic device may spray less amount of the aerosolized flavor material at the timepoint of the puff of the user. Depending on the puff interval of the user, the processor 100 may use both or one of the two adjustment methods described above.

For example, when the preset first interval threshold is 8 seconds and the puff interval of the user is 5 seconds, the processor 100 may spray once every 10 seconds by spraying once at the user's puff and not spraying for the following puff. Alternatively, the processor 100 may decrease the amount of the aerosolized flavor material sprayed at every user's puff by half and may spray the aerosolized flavor material. The number of sprays or the spray amount are not limited thereto.

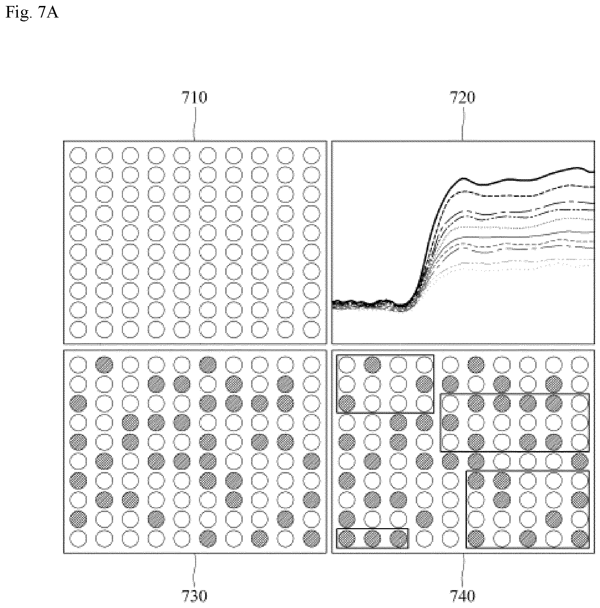

FIG. 7A schematically illustrates an operation of a flavor sensor of an electronic device according to one embodiment.

The descriptions provided with reference to FIGS. 6A to 6C may be applicable to FIG. 7A and any repeated description related thereto may be omitted. The processor 100 of FIG. 1A may perform an operation to analyze data of a flavor sensor.

The flavor sensor in one embodiment may include one or more samples inside the flavor sensor to sense the flavor of a substance. The samples may react to components of the flavor of the substance to detect which components are included in the flavor of the substance. The samples may include a plurality of components predetermined inside the flavor sensor.

An initial state 710 of the samples in one embodiment may be a state in no reaction with any component. In the initial state 710, an electronic device may operate the flavor sensor to sense flavor. When the flavor sensor operates, the flavor of the substance may flow into the flavor sensor and may react with the sample. Although the example of the present disclosure includes 100 samples, the number of samples is not limited to 100 and may be more or less.

A sample data extraction graph 720 in one embodiment may represent the reaction of a component of the flavor of the substance as a graph. Each sample may react to a component of the flavor of the substance and data may be extracted by a processor of the electronic device. The data extraction graph 720 may express components of the flavor of the substance and concentrations thereof. For example, a line with the highest peak in the graph 720 may be a component showing the greatest reactivity, and thus, may have the highest concentration. On the other hand, a line with the lowest peak in the graph 720 may be a component showing the smallest reactivity, and thus, may have the lowest concentration.

A reaction state 730 of the samples in one embodiment may represent samples after reacting to the components of the flavor of the substance from the initial state 710. Based on the graph 720 and the reaction state 730, the flavor sensor may store the component and the concentration value of the flavor of the substance.

The processor in one embodiment may convert flavor data into a flavor recipe based on the information of a flavor diffusing cartridge including a plurality of cartridges. Referring to the flavor cartridge unit 600 of FIG. 6A as an example, the processor 100 may respectively map a plurality of predetermined components (samples) onto the plurality of cartridges (e.g., the flavor cartridges 611 to 613), based on information of the three cartridges 611 to 613. A diagram 740 may be a diagram illustrating a result of mapping the reaction state 730 of the samples onto the plurality of cartridges.

When mapping is completed, the processor may determine a mixing ratio of flavor materials based on an average value of concentrations of the components. Alternatively, the processor may determine the mixing ratio of flavor materials based on a concentration value of a component that is the most similar to a flavor cartridge among the components. Other than the method described in the present disclosure, a mixing ratio of flavor materials may be determined and when the flavor cartridge unit 600 includes flavor cartridges more than three, the processor 100 may map samples to a level that is similar to the actual flavor of the substance.

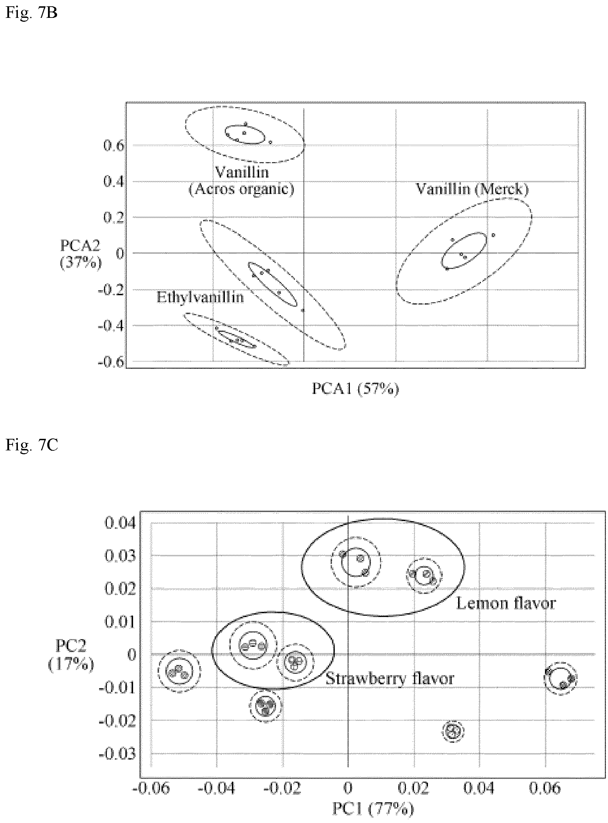

FIG. 7B illustrates a data distribution diagram based on a flavor analyzed by an electronic device according to one embodiment.

Referring to FIG. 7B, an electronic device in one embodiment may analyze a principal component of multiple flavor materials. The electronic device may detect data distribution through a principal component analysis (PCA). The electronic device may build a library with the data distribution and may store the data distribution as data.

FIG. 7B is an analysis example of vanillin in one embodiment. Vanillin may have different composition ratios depending on the manufacturer. The electronic device may accumulate data on a flavor by analyzing a principal component constituting the flavor.

The graph of FIG. 7B may represent an analysis result showing two representative principal components constituting vanillin as the x-axis (PCA1) and the y-axis (PCA2). The 0 point of the x-axis may be a point in which PCA1 (e.g., a chemical substance A) has a composition ratio of 57% and the 0 point of the y-axis may be a point in which PCA2 (e.g., a chemical substance B) has a composition ratio of 37%. That is, PCA1 and PCA2 may represent concentration values of principal components, respectively, and may be a two-dimensional representation of two substances in the graph. On the x-axis, PCA1 (57%) may represent that a component value of the chemical substance A differs its concentration value by -0.5 and +0.5 based on the 57% point (e.g., a point where Acros organic vanillin is distinguished from Merck vanillin in the diagram). On the y-axis, PCA2 (37%) may represent that a component value of the chemical substance B differs its concentration value by -0.2 and +0.2 (e.g., a point where Ethylvanillin is distinguished from Acros organic vanillin in the diagram).

For example, when measuring vanillins from different manufacturers five times, composition ratios of principal components of Merck vanillin, Acros organic vanillin, and Ethylvanilin may be clustered in specific areas as shown in FIG. 7B. The electronic device may store the clustered data and may improve analysis accuracy on the substance when sensing the flavor in the future. However, the present disclosure is not limited to the method of detecting vanillin and may be applicable to a detection method of various flavor materials in a market.

FIG. 7C illustrates an example of showing data of a sensing result of a flavor material by an electronic device according to one embodiment.

The graph of FIG. 7C is provided based on the same method as the graph of FIG. 7B. A composition ratio of a principal component may be expressed by a two-dimensional (2D) graph where the x-axis is based on a point in which the concentration of a principal component PC1 is 77% and the y-axis is based on a point in which the concentration of a principal component PC2 is 17%.

Referring to FIG. 7C, the electronic device in one embodiment may analyze a component of a flavor material. A method of an electronic device to identify a flavor material may use data stored and built as a library by sensing various flavor materials in advance. The electronic device may sense a specific flavor material and may identify a flavor material by matching a sensing value of the specific flavor material with the library data.

The graph of FIG. 7C illustrates a data distribution generated by sensing various flavor materials, building a library, and storing in advance for two principal components. More specifically, data sensing and clustering various flavor materials, including lemon flavor and strawberry flavor, is illustrated.

For example, the graph of FIG. 7C may express, as a graph, a method of matching lemon flavor and strawberry flavor with data generated by sensing various flavor materials and building a library in advance. The lemon flavor may be already built as a library and stored in an upper end of the graph of FIG. 7C and when the electronic device detects that a result value of sensing an external flavor material is in an area having a value stored as the lemon flavor, the electronic device may determine that the external flavor material is the lemon flavor. Similarly, the strawberry flavor may be already built as a library and stored in the left side of the graph of FIG. 7C and when the electronic device detects that a result value of sensing another external flavor material is in an area having a value stored as the strawberry flavor, the electronic device may determine that the other external flavor material is the strawberry flavor.

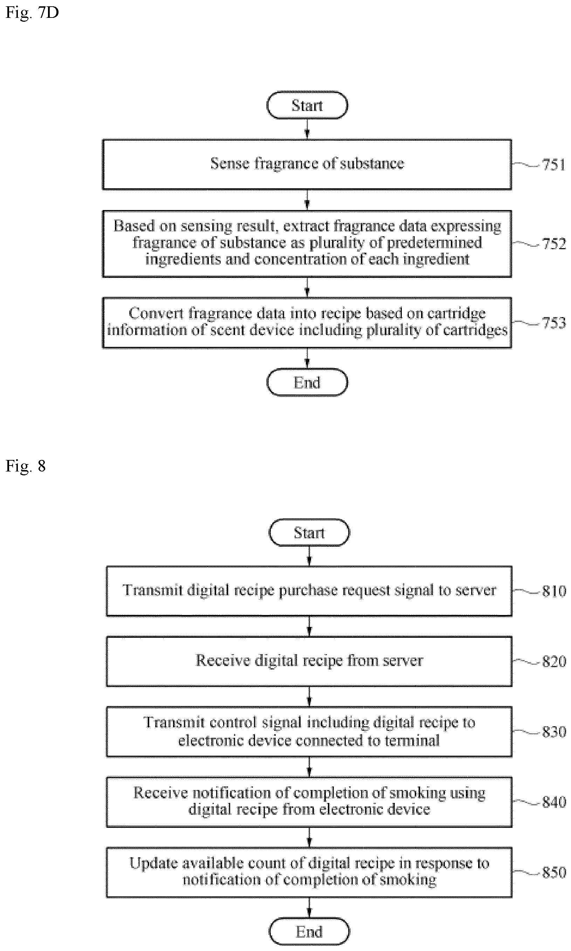

FIG. 7D illustrates a flowchart of an electronic device to generate a flavor recipe according to one embodiment.

Referring to FIG. 7D, operations 751 to 753 may be performed by the processor 100 and the flavor sensor of the electronic device described with reference to FIGS. 1A to 7C and a repeated description thereof may be omitted.

In operation 751, an electronic device in one embodiment may sense the flavor of a substance.

The flavor of the substance may enter into a flavor sensor of the electronic device in one embodiment. A plurality of predetermined components in the flavor sensor may react to a component of the flavor of the substance. Data on the plurality of reacted predetermined components may be transmitted to the processor 100.

In operation 752, the electronic device may extract flavor data expressing the flavor of the substance by the plurality of predetermined components and concentrations of the components, based on the sensing result.

The processor 100 in one embodiment may extract flavor data expressing types and concentrations of components of the flavor of the substance by analyzing the plurality of reacted predetermined components. For example, when the number of components of the flavor of the substance is 10, the processor 100 may quantify and store types and concentrations of 10 components.

In operation 573, the flavor data may be converted into a recipe based on cartridge information of a flavor diffusing device including a plurality of cartridges.

The processor 100 in one embodiment may convert the flavor data into a recipe based on cartridge information of the flavor diffusing device including a plurality of cartridges. The cartridge information may include at least one of the number and type of the plurality of flavor cartridges. The processor 100 may map the plurality of predetermined components in the flavor sensor onto the plurality of flavor cartridges, respectively, based on the cartridge information. That is, based on the flavor cartridge information, the processor 100 may set similar components to be a plurality of flavor component groups among the plurality of predetermined components and may map the flavor cartridges onto the flavor component groups, respectively.

The processor 100 in one embodiment may set spray amounts of the plurality of flavor cartridges based on the respective concentrations of the components. That is, the processor 100 may determine a mixing ratio of flavor cartridges of a flavor diffusing cartridge based on the concentration data of an individual component in the mapped flavor component group. For example, it may be supposed that three components of a first flavor component group react to the flavor of a substance, two components of a second flavor component group react to the flavor of the substance, and five components of a third flavor component group react to the flavor of the substance. In this case, by comparing the concentration of three components of the first flavor component group, the concentration of two components of the second flavor component group, and the concentration of five components of the third flavor component group, the respective concentrations of the flavor component groups may be determined. The processor 100 may determine a spray amount (e.g., a mixing ratio) of the flavor cartridges based on each concentration of the flavor component group. Consequently, the processor 100 may generate a flavor recipe including the flavor data, the cartridge information of the flavor diffusing device, and the mixing ratio.

The flavor cartridge unit 600 in one embodiment may mix the plurality of flavor materials based on the generated flavor recipe and may provide an aerosolized flavor material.

FIG. 8 illustrates a flowchart of an operating method of a terminal according to one embodiment.

In operation 810, a terminal in one embodiment may transmit a digital recipe purchase request to a server. The user may access the server through a terminal on which an application is installed. The server may serve as a service platform for providing a digital recipe provision service. The user may subscribe to the digital recipe provision service provided by the server through the application and may generate an account of the digital recipe provision service. The server may be connected to the terminal through a network. In this case, the network may include the Internet, at least one local area network, a wide area network, a cellular network, a mobile network, and other networks, or a combination thereof.

For example, in a smartphone application for purchasing a digital recipe for a cigarette, various recipes corresponding to various cigarettes (e.g., Raison, Esse, Marlboro, and the like) may be listed. The user may purchase a recipe related to a cigarette that the user desires to smoke in the application. That is, the user may transmit a request signal to purchase a digital recipe of a predetermined cigarette to the server to receive the digital recipe. As described with reference to FIGS. 1 to 7D, the digital recipe may include a digital taste recipe that is information of a current applied to an object contacting an electronic device. In addition, the digital recipe may include a digital flavor recipe that is information on a method of diffusing flavor of a diffusing flavor cartridge of the electronic device.

In operation 820, the terminal in one embodiment may receive the digital recipe from the server.

For example, the terminal may transmit a purchase request signal for a digital recipe of a cigarette to the server and the server may transmit the digital recipe to the terminal in response to the purchase request signal of the terminal. The terminal may download and store the received digital recipe in the terminal.

In operation 830, the terminal in one embodiment may transmit a control signal including the digital recipe to the electronic device.

According to one embodiment, the terminal and the electronic device may be connected via at least one wireless communication of short-range communication or cellular communication.

The terminal in one embodiment may receive the digital recipe from the server and may store the digital recipe in the terminal. The terminal may transmit, to the electronic device, a control signal for operating the electronic device, such as a power on or off signal and a preheating signal, as well as the digital recipe of the cigarette. In this case, the control signal may include information on a maximum puffing count available for the user to puff for a single time smoking. In addition, the digital recipe may include information on electrical stimulation or thermal stimulation implementing the digital taste.

In operation 840, the terminal in one embodiment may receive a notification of the completion of smoking using the digital recipe from the electronic device.

For example, when the user finishes smoking with the digital recipe using the electronic device, the electronic device may transmit, to the terminal, a notification of the completion of smoking by the user. In this case, the electronic device may transmit, to the terminal, a notification of the completion of smoking determined based on the maximum puffing count available for one time smoking of the user wherein the maximum puffing count is included in the control signal received from the terminal.

In operation 850, the terminal in one embodiment may update an available use count of the digital recipe in response to the notification of the completion of smoking.

For example, when the terminal receives the notification of the completion of smoking based on the digital recipe from the electronic device, the terminal may decrease the available use count of the digital recipe by one. For example, since a typical pack of cigarettes includes 20 cigarettes, typically, the digital recipe of the cigarette may also have an available use count of 20. In this case, when the user completes smoking and the terminal receives the notification of the completion of smoking, the terminal may update a use count to 19 by decreasing the use count by one.

When the available use count of the digital recipe is 0, the terminal may deactivate the digital recipe.

For example, when a use count of the digital recipe reaches 0 by using 20 times that is the use count of the digital recipe, the terminal may transmit a signal that the digital recipe is exhausted to the server.

The terminal in one embodiment may transmit a deactivation signal of the digital recipe to the electronic device.

As described above, when the use count of the digital recipe reaches 0, a notification that smoking using the digital recipe is no longer available may be sent by transmitting the deactivation signal to the electronic device.

The terminal in one embodiment may present a use pass for using a digital recipe a predetermined number of times to another terminal. For example, because transmission and reception between users through a server using a smartphone application is possible, a digital recipe purchased by a user may be presented to another user. In this case, in the terminal of the user who presents, the use count of the digital recipe that is presented may be deducted (e.g., if the user presents 3 times, the use count is deducted by 3).

The terminal in one embodiment may transmit a subscription signal to the server.

For example, when the smartphone application provides a subscription service, the terminal may transmit a signal to subscribe to the server. The subscription service may automatically repurchase the digital recipe based on the number of uses of the digital recipe by the user. Alternatively, when a predetermined amount is paid in advance, it may allow the use of various cigarettes without a limitation on the number of uses.

The terminal in one embodiment may receive usage information including at least one of the number of available digital recipes corresponding to the subscription signal, a maximum available use count of the digital recipe, and a maximum puffing count of the digital recipe.

For example, when the user starts to use a subscription service via a smartphone application, the terminal may transmit a signal that the user uses the subscription service to the server. In this case, the server may transmit the number of available digital cigarette recipes depending on the type of subscription service selected by the user. Alternatively, the server may transmit usage information including at least one of the available use count of the digital cigarette recipe and the maximum puffing count of the digital cigarette recipe. The terminal may provide a subscription service to the user based on the signal transmitted by the server.

According to one embodiment, the subscription service may recommend a personalized recipe by analyzing accumulated data, such as the smoking pattern of the user and purchase information, and may provide new recipe information. The subscription service may be a monthly fixed-rate system and may provide differentiated benefits by grade. For example, the subscription service may provide a user with a differentiated grade, such as a VIP service, a gold service, and a silver service. Depending on the grade of the user, the number of recipe downloads (e.g., unlimited for VIPs), the number of recipe gifts, and a flavor cartridge delivery service provided to the user may vary. However, the type of grades and type of services described herein are not limited thereto.

The electronic device in one embodiment may receive the control signal including the digital recipe from the terminal connected to the electronic device. The control signal may include the digital recipe as well as a maximum puffing count for one time smoking.

The electronic device in one embodiment may count the number of puffs of an object for one time smoking based on the control signal.

For example, when the terminal sets the maximum puffing count to 14 times and transmits it to the electronic device, the puff sensor of the electronic device may sense puffs of the user and may decrease the puffing count.

When the puffing count of the object in one embodiment reaches the maximum puffing count, a notification of the completion of smoking using the digital recipe may be sent to the terminal.

For example, when the maximum puffing count is 14 and the user completes 14 times of puffing, the electronic device may transmit a notification of completion of smoking to the terminal.

The digital recipe in one embodiment may be sold as a do-it-yourself (DIY) type, a cigarette type, a time, place, and occasion (TPO) type, or a customer-made type.

For example, the DIY type may be a type in which a consumer directly customizes an amount of nicotine, the taste or flavor of a cigarette, and generates a recipe. For example, through the DIY type, the user may select at least one of nicotine amounts (e.g., 0.1 mg and 0.3 mg) and may select wine as the taste of the cigarette and may select an herbal flavor as the flavor of the cigarette.

For example, the cigarette type may allow the consumer to select a taste or flavor similar to the typical cigarette to be the digital recipe. That is, the taste of the cigarette currently sold on the market may be sold in the form of a digital recipe.

For example, the TPO type may allow the user to select a recipe suitable for a smoking circumstance. For example, because the taste of a cigarette felt by a smoker may vary while driving, taking a rest, or after drinking or eating, the user may select the cigarette taste that is suitable for a circumstance.

For example, the customer-made type may allow the consumer who owns the electronic device to create a recipe using the flavor sensor. The created recipe may be sold to other consumers through a smartphone application.

However, the present disclosure is not limited to the examples described above and the digital recipe may be generated with other tastes or flavors in the market, the user may select other tastes or flavors, or other types may be sold.

FIG. 9A schematically illustrates a configuration of an electronic device according to one embodiment.

The descriptions provided with reference to FIGS. 1A to 8 may be applicable to FIG. 9A and any repeated description related thereto may be omitted.

An electronic device 900 in one embodiment may include a mouthpiece unit 910, a sensing unit 921, a battery 922, a processor 923, a communication unit 924, a flavor sensor unit 931, a flavor cartridge unit 932, and a liquid cartridge unit 933.

The mouthpiece unit 910 in one embodiment may include the same function and structure as the mouthpiece unit 110 of FIG. 1A and the mouthpiece unit 400 of FIG. 4A described above.

The sensing unit 921 may sense a state of the electronic device 900 or a state around the electronic device 900 and may transmit the information obtained by sensing to the processor 923.

The sensing unit 921 may include at least one of a temperature sensor, an insertion detection sensor, or a puff sensor, but is not limited thereto. For example, the sensing unit 921 may include sensors described with reference to the drawings. The temperature sensor may sense a temperature to prevent a heating wire unit of the mouthpiece unit 910 from overheating and may transmit the temperature to the processor 923.

The insertion detection sensor may sense the insertion and/or removal of the mouthpiece unit 910 from the electronic device 900. The insertion detection sensor may include, for example, at least one of a film sensor, a pressure sensor, a light sensor, a resistive sensor, a capacitive sensor, an inductive sensor, or an infrared sensor, which may sense a signal change by the insertion and/or removal of the mouthpiece unit 910.

The sensing unit 921 may further include at least one of a temperature/humidity sensor, an atmospheric pressure sensor, a magnetic sensor, an acceleration sensor, a gyroscope sensor, a position sensor (e.g., a global positioning system (GPS)), a proximity sensor, and a red, green, blue (RGB) sensor (e.g., an illuminance sensor), in addition to the sensors described above. A function of each sensor may be intuitively inferable from its name by those having ordinary skill in the art, and thus a more detailed description thereof will be omitted here.

The battery 922 may supply power to be used to operate the electronic device 900. The battery 922 may supply power to heat the liquid cartridge unit 933 or the flavor cartridge unit 932. In addition, the battery 922 may supply the power required for the operations of other components provided in the electronic device described above. The battery 922 may be a rechargeable battery or a disposable battery. For example, the battery 922 may be a lithium polymer (LiPoly) battery.

The processor 923 may control the overall operation of the electronic device 900. In one embodiment, the processor 923 may be implemented as an array of a plurality of logic gates or may be implemented as a combination of a general-purpose microprocessor and a memory in which a program executable by the microprocessor is stored. In addition, it is to be understood by one of ordinary skill in the art to which the disclosure pertains that the processor may be implemented in other types of hardware.

The processor 923 may perform operations of the processor 100 described with reference to FIGS. 1A to 8.

The communication unit 924 may include at least one component for communicating with another electronic device. For example, the communication unit 924 may include a short-range wireless communication unit and a wireless communication unit.

The short-range wireless communication unit may include a Bluetooth communication unit, a Bluetooth low energy (BLE) communication unit, a near-field communication unit, a WLAN (Wi-Fi) communication unit, a ZigBee communication unit, an infrared data association (IrDA) communication unit, a Wi-Fi direct (WFD) communication unit, an ultra-wideband (UWB) communication unit, and an Ant+ communication unit, but is not limited thereto.

The wireless communication unit may include, for example, a cellular network communicator, an Internet communicator, a computer network (e.g., a local area network (LAN) or a wide-area network (WAN)) communicator, and the like, but is not limited thereto. The wireless communication unit 924 may use subscriber information (e.g., international mobile subscriber identity (IMSI)) to identify and authenticate the electronic device 900 in a communication network.

The communication unit 924 may transmit a notification of the completion of smoking to a terminal connected to the electronic device.

The flavor sensor unit 931 may include the flavor sensor of the electronic device described with reference to FIGS. 7A to 7D.

The flavor cartridge unit 932 may include the flavor cartridge unit of the electronic device described with reference to FIGS. 6A to 6C.

The liquid cartridge unit 933 may include a nicotine medium or a non-nicotine medium. The liquid cartridge unit 933 may vaporize and provide liquid to a user. Information on the nicotine medium or the non-nicotine medium may be included in a digital recipe. Accordingly, the electronic device may provide a medium in the liquid cartridge unit 933 based on the digital recipe and may provide the medium to the user. The nicotine medium may include a substance (e.g., leaf tobacco, nicotine gel, nicotine-activated carbon, nicotine-containing paraffin wax, a tobacco medium compression stick, and the like) that is able to provide nicotine. A non-nicotine substance may be health care food and may include ginseng, caffeine, taurine, vitamin, and the like. The electronic device may heat and aerosolize the non-nicotine substance and may allow the user to inhale. Various heating methods may be allowed, such as induction heating, external heating, internal heating, and ultrasonic heating.

FIG. 9B illustrates a flowchart of an operating method of an electronic device as a digital cigarette according to one embodiment.

Operations 941 to 943 may be performed by the processor described with reference to FIGS. 1A to 9A.

In operation 941, an electronic device in one embodiment may receive information on digital taste and digital flavor from a terminal connected to the electronic device.

In operation 942, the electronic device in one embodiment may generate a taste control signal corresponding to the digital taste information and a flavor control signal corresponding to the digital flavor information.

In operation 943, the electronic device in one embodiment may generate digital taste and digital flavor based on the digital taste control signal and the digital flavor control signal.

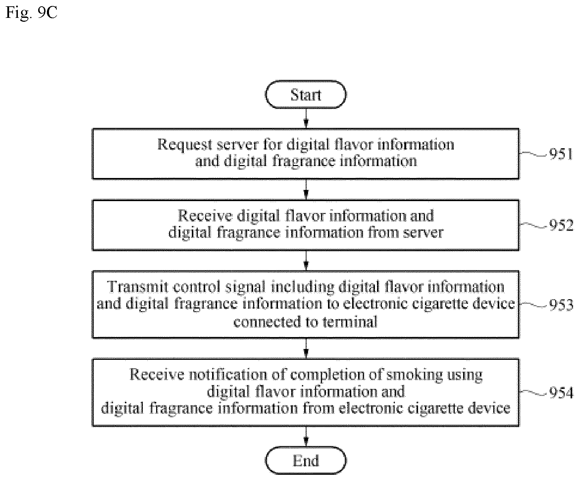

FIG. 9C illustrates a flowchart of an operating method of a terminal for a digital cigarette according to one embodiment.

Operations 951 to 954 may be performed by the processor described with reference to FIGS. 1A to 9A.

In operation 951, a terminal in one embodiment may send a request to a server for the digital taste information and the digital flavor information.

In operation 952, the terminal in one embodiment may receive the digital taste information and the digital flavor information from the server.

In operation 953, the terminal in one embodiment may transmit a control signal including the digital taste information and the digital flavor information to the electronic device connected to the terminal.

In operation 954, the terminal in one embodiment may receive a notification of completion of smoking using the digital taste information and the digital flavor information from the electronic device.

The electronic device, the method of controlling the electronic device, the terminal, and the method of controlling the terminal described with reference to FIGS. 1A to 9A may be included in the operating method of FIGS. 9B and 9C.

The methods according to the above-described examples may be recorded in non-transitory computer-readable media including program instructions to implement various operations of the above-described examples. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape, optical media such as CD-ROM discs, DVDs, and/or Blue-ray discs, magneto-optical media such as optical discs, and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter. The above-described devices may be configured to act as one or more software modules in order to perform the operations of the above-described example embodiments, or vice versa.

The software may include a computer program, a piece of code, an instruction, or some combination thereof, to independently or uniformly instruct or configure the processing device to operate as desired. Software and data may be embodied permanently or temporarily in any type of machine, component, physical or pseudo equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

A number of example embodiments have been described above. Nevertheless, it should be understood that various modifications may be made to these example embodiments. For example, suitable results may be achieved if the described techniques are performed in a different order and/or if components in a described system, architecture, device, or circuit are combined in a different manner and/or replaced or supplemented by other components or their equivalents.

Accordingly, other implementations are within the scope of the following claims.

## Claims

1. A method of controlling an electronic device, the method comprising:
receiving a digital recipe;
determining whether a first area of a mouthpiece unit is in contact with a first portion of an object;
determining whether a second area of the mouthpiece unit is in contact with a second portion of the object;
determining whether a first terminal and a second terminal of the mouthpiece unit are in contact with a third portion of the object; and
based on the contact of the first terminal and the second terminal of the mouthpiece unit with the third portion of the object, applying a current corresponding to the digital recipe to the third portion of the object.

2. The method of claim 1, further comprising:
heating a nicotine medium and a heating wire of the mouthpiece unit based on the received digital recipe.

3. The method of claim 1, wherein the determining of the contact of the first portion of the object comprises determining whether a capacitance value of the first area of the mouthpiece unit exceeds a first capacitance threshold value, and
the determining of the contact of the second portion of the object comprises determining whether a capacitance value of the second area of the mouthpiece unit exceeds a second capacitance threshold value.

4. The method of claim 1, wherein the determining of the contact of the third portion of the object comprises:
measuring a voltage of the second terminal of the mouthpiece unit; and
when the measured voltage exceeds a third voltage threshold value, determining that the mouthpiece unit is in contact with the third portion of the object.

5. The method of claim 1, wherein the applying of the current to the third portion of the object comprises:
applying the current to the third portion of the object only when the first terminal and the second terminal of the mouthpiece unit are in contact with the third portion of the object.

6. A non-transitory computer-readable storage medium storing instructions that, when executed by a processor, cause the processor to perform the method of claim 1.

7. An electronic device comprising:
a main body;
a mouthpiece unit detachable from the main body;
wherein the main body comprises:
a communication unit configured to receive a digital recipe; and
a processor configured to:
determine whether a first area of the mouthpiece unit is in contact with a first portion of an object,
determine whether a second area of the mouthpiece unit is in contact with a second portion of the object,
determine whether a first terminal and a second terminal of the mouthpiece unit are in contact with a third portion of the object, and
based on the contact of the first terminal and the second terminal of the mouthpiece unit with the third portion of the object, apply a current corresponding to the digital recipe to the third portion of the object.

8. The electronic device of claim 7, wherein the mouthpiece unit comprises an aerosol intake through which aerosol inhaled by the object passes.

9. The electronic device of claim 7, further comprising:
a first terminal and a second terminal in a third area of the electronic device; and
a heating wire unit comprising a heating wire disposed in a third area of the mouthpiece unit and heating the mouthpiece unit, based on the digital recipe.
